Europäisches Patentamt

European Patent Office

Office européen des brevets

(11) Numéro de publication: **0 364 367 B1**

(12) **FASCICULE DE BREVET EUROPEEN**

(45) Date de publication de fascicule du brevet: **22.06.94**　(51) Int. Cl.⁵: **A61L 2/18**, A01N 59/00

(21) Numéro de dépôt: **89402843.0**

(22) Date de dépôt: **13.10.89**

(54) **Procédé pour nettoyer et/ou désinfecter et stériliser des appareils d'hémodyalise.**

(30) Priorité: **13.10.88 FR 8813497**

(43) Date de publication de la demande:
**18.04.90 Bulletin  90/16**

(45) Mention de la délivrance du brevet:
**22.06.94 Bulletin  94/25**

(84) Etats contractants désignés:
**BE CH DE ES GB IT LI NL SE**

(56) Documents cités:
**EP-A- 0 207 633**
**GB-A- 1 571 975**

(73) Titulaire: **LABORATORIOS INIBSA S.A.**
**Loreto 10**
**08029 Barcelone(ES)**

(72) Inventeur: **Riera Aixala, José**
**Ingenerio 11-13**
**E-08400 Granollers(ES)**

(74) Mandataire: **Durand, Yves Armand Louis et al**
**Cabinet Z. Weinstein**
**20, Avenue de Friedland**
**F-75008 Paris (FR)**

EP 0 364 367 B1

## Description

La présente invention concerne le nettoyage et/ou la désinfection et la stérilisation des appareils d'hémodialyse.

Ces appareils sont fréquemment utilisés dans les hôpitaux pour le traitement des malades atteints d'insuffisance rénale aiguë ou chronique. En effet, ces malades sont soumis à un système d'épuration du sang appelé hémodialyse qui consiste à maintenir l'équilibre ionique du sang, éliminer les produits résultant du métabolisme, principalement des protéines, et maintenir l'équilibre hydrique de l'organisme.

Ceci se réalise au moyen de l'hémodialyse qui comprend une membrane semi-perméable qui sépare deux circuits, l'un, où circule le sang du patient, l'autre, où circule le liquide de dialyse qui est une solution à base d'une composition électrolytique identique à celle du liquide intercellulaire. La membrane de cet hémodialyseur permet le passage de substances d'une taille moléculaire petite et moyenne jusqu'à des masses moléculaires de 10 à 40 000 daltons.

Le fonctionnement de l'hémodialyseur est contrôlé par un appareil dénommé dispositif de contrôle de l'hémodialyse ou encore plus couramment rein artificiel. Ce dernier a également pour fonction la fabrication du liquide de dialyse, constitué par une solution de sels de sodium, de potassium, de calcium et de magnésium, additionnée d'une quantité importante de glucose. Ce liquide est chauffé à 37°C puis passe par l'hémodialyseur où il reçoit des substances organiques qui proviennent du sang à travers la membrane. Ces substances organiques, d'une part, et inorganiques comme des sels de calcium et de magnésium, d'autre part, se déposent ou précipitent sur les surfaces internes des circuits du dispositif de contrôle de l'hémodialyse.

Par ailleurs, au cours des manipulations, des petites fuites peuvent se produire et contaminer la machine. Ceci entraîne un risque important de contamination du patient et du personnel utilisant ce matériel.

Pour ces raisons, les appareils d'hémodialyse doivent être désinfectés après chaque traitement pour éliminer la contamination bactérienne qui se développe à la suite de conditions propices d'humidité, de température, de temps et en présence de substances nutritives. De plus, compte tenu de la présence de germes dans les circuits, il est important de désinfecter vu le risque important que représente une infection par les micro-organismes.

De ce fait, le nettoyage et/ou la désinfection et la stérilisation d'un appareil d'hémodialyse doivent répondre aux exigences suivantes : éliminer les barrières de type mécanique qui empêchent le contact intime entre le produit désinfectant et les germes, et éliminer les barrières de type chimique qui diminuent l'effet du produit désinfectant.

Ceci se traduit par l'utilisation d'un produit de nettoyage et/ou de désinfection et de stérilisation qui a une activité bactéricide, sporicide et virucide, qui disperse les dépôts organiques qui se forment sur les surfaces internes du circuit, qui dissout les dépôts formés par les précipités inorganiques, qui désinfecte en phase gazeuse afin que ce produit soit efficace sur les surfaces en contact avec la vapeur sans mouiller celles-ci, et enfin qui est compatible avec les matériaux constituant les appareils d'hémodialyse.

Or, traditionnellement, les appareils d'hémodialyse sont nettoyés et désinfectés avec de l'hypochlorite sodique (sel de l'acide hypochloreux) à 5,25% dilué dans de l'eau du réseau à 1:35. Un tel procédé se réalise en conditions alcalines avec un pH entre 11 et 12 ou supérieur. De ce fait, un grand nombre de composés lipides ou protéiniques (le plus souvent des polypeptides de faibles poids moléculaire) tels que l'urée, la créatinine, les hydroxydes alcalins restant en tant que résidu d'une hémodialyse précédente, précipitent. En effet, ces composés ainsi que les bactéries et les virus sont passés à travers la membrane semi-perméable du dialyseur, du sang du malade au dispositif d'hémodialyse. Cette opération de nettoyage est répétée. Par conséquent, l'incrustation de dépôts augmente avec le nombre de traitement tout en étant de plus facilitée par un environnement approprié aux micro-organismes comme l'emplacement et la porosité des surfaces dans les appareils. Des infections croisées peuvent même être engendrées dans ce cas.

Au bout de deux semaines d'utilisation ininterrompue de l'appareil de dialyse, à raison de 3 séances journalières de dialyse de 3 ou 4 heures par séance, avec un nettoyage-désinfection entre chaque séance (en passant d'un patient à l'autre), l'incrustation est telle qu'il est nécessaire de recourir à un nettoyage plus efficace. A ce stade, des solutions acides sont utilisées pour éliminer les dépôts incrustés. En général, ces solutions sont à base d'acide oxalique ou d'un mélange d'acide oxalique et d'acide acétique. Cet acide oxalique est utilisé pour ses propriétés dissolvantes des incrustations et pour ses propriétés antichlore et antirouille ainsi que pour son absence d'activité corrosive sur l'acier inoxydable, le nylon, la gomme de silicone, le téflon et le verre, composants habituels de ces appareils. Mais, lorsque cette phase de désincrustation est terminée, toute trace d'acide doit être éliminée. En effet, l'acide oxalique étant toxique, est dangereux pour le patient suivant qui subit la dialyse.

Un lavage est alors effectué avec une solution de bicarbonate qui est neutralisante.

Cependant, malgré la phase de désincrustation suivie du lavage neutralisant, il est difficile d'éliminer toutes les traces de virus et de bactérie qui peuvent subsister dans les parties les plus inaccessibles des appareils d'hémodialyse, telles que les jauges ou les joints des différentes valvules.

Dans ces conditions, un dernier traitement est requis :
une stérilisation au formol tous les mois ou mois et demi.

Ce traitement s'effectue en phase vapeur où il est possible d'atteindre les endroits invisibles ou les endroits où le liquide n'agit pas à cause de l'air. En général, les opérateurs ont recours au formol pour éliminer une contamination par Pseudomonas Aeruginosa.

Ce processus de nettoyage nécessite un arrêt de l'appareil pendant au moins 24 heures. En outre, il n'est pas exclu que des traces de formol subsistent dans l'appareil, ce qui n'est pas sans danger vu le caractère mutagène du formol.

Il est décrit dans le brevet européen EP-0 207 633 de Tell et al des compositions stérilisantes de nettoyage et de stérilisation d'appareils d'hémodialyse comprenant du chlorite de sodium, de l'acide citrique et un tampon de façon à former une solution isotonique avec un pH d'approximativement 7,3. La présence du tampon permet de maintenir le pH à approximativement 7,3 ce qui permet de ne pas rompre la couche de protéines qui se forme sur la surface interne des fibres poreuses de la membrane de dialyse. Ceci en vue d'une réutilisation de la membrane de dialyse.

Par ailleurs, le brevet GB-571 975 de Alliger décrit des compositions acides pour nettoyer des appareils chirurgicaux mais ne concerne pas le nettoyage d'appareils d'hémodialyse.

La présente invention a alors pour objet un procédé de nettoyage et/ou de désinfection et de stérilisation des appareils d'hémodialyse qui permet à la fois d'éliminer les dépôts organiques et les précipités inorganiques tout en ayant une activité bactéricide, sporicide et virucide et une activité désinfectante en phase gazeuse.

Le procédé selon l'invention consiste à :
- mélanger une composition basique comprenant l'ion chlorite avec une composition acide comprenant de l'acide lactique dans un rapport de la composition acide à la composition basique allant de 1:2 à 3:4,
- introduire le mélange ainsi obtenu additionné d'eau dans un appareil d'hémodialyse, le rapport du mélange à l'eau étant d'environ 1:34, pour former une solution finale acide, et
- faire circuler la solution finale dans l'appareil où le mélange est dilué dans l'eau et où la composition basique réagit avec la composition acide selon les formules suivantes :

$$CH_3\text{-}CH(OH)\text{-}COOH + ClO_2{}^- \rightarrow HClO_2 + CH_3\text{-}CH(OH)\text{-}COO^-$$

et

$$5HClO_2 \rightarrow 4ClO_2 + HCl + 2H_2O$$

Selon une caractéristique de l'invention, la composition basique contient de 30 à 50% en masse de chlorite de sodium et de 70 à 50% en masse d'eau déïonisée.

Selon une autre caractéristique de l'invention la composition acide contient 70 à 90% en masse d'acide lactique et 30 à 10% en masse d'eau déïonisée.

Selon encore une autre caractéristique de l'invention le mélange est introduit en continu ou en une seule fois dans l'appareil d'hémodialyse.

Selon une dernière caractéristique de l'invention la solution finale circule pendant 10 minutes dans l'appareil d'hémodialyse.

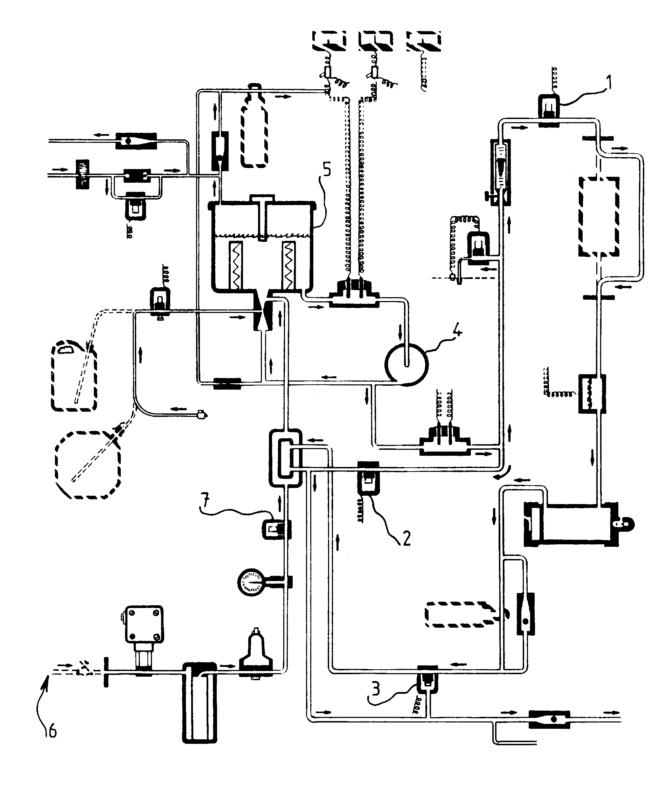

L'invention sera mieux comprise, et d'autres buts, caractéristiques et détails de celle-ci apparaîtront plus clairement à la lecture de la description explicative qui va suivre faite en référence à la figure unique annexée donnée uniquement à titre d'exemple non limitatif.

Cette figure est une représentation schématique du dispositif d'hémodialyse en phase de rinçage.

Le procédé de nettoyage et/ou de désinfection et de stérilisation selon l'invention a pour objet l'utilisation d'une solution libérant du dioxyde de chlore comme produit désinfectant, au sein du circuit du dispositif d'hémodyalise.

Le milieu de nettoyage et/ou de désinfection devient alors acide avec un pH allant de 2,8 à 3,2.

Comme le dioxyde de chlore à températures normales s'évapore rapidement et est difficile à contrôler, il est impossible de l'utiliser directement.

De même à l'état de gaz concentré, il est explosif et nocif.

Par conséquent, le procédé selon l'invention utilisant le dioxyde de chlore, s'effectue en partant d'une composition basique comprenant du chlorite sodique qui libérera, au cours d'une réaction, le dioxyde de chlore in situ dans l'appareil d'hémodialyse.

Plus précisément, le procédé comprend les étapes suivantes :

- préparation d'une composition basique ou solution base à partir de 30 à 50% en masse de chlorite sodique et 70 à 50% en masse d'eau déïonisée,
- préparation d'une composition acide ou solution activatrice à partir de 70 à 90% en masse d'acide lactique et 30 à 10% en masse d'eau déïonisée,
- mélange de la solution base avec la solution activatrice dans une proportion de la solution activatrice à la solution base allant de 1:2 à 4:4 et de préférence 3:4,
- dilution du mélange obtenu à l'étape précédente dans de l'eau courante dans un rapport du mélange à l'eau d'environ 1:34, soit une dilution de 1/35, et
- faire circuler le mélange en cours de dilution dans l'eau, dans l'appareil d'hémodyalise.

La réaction en deux temps qui a lieu dans l'appareil est la suivante :

$$CH_3\text{-}CH(OH)\text{-}COOH \ + \ ClO_2Na \rightarrow HClO_2 \ + \ CH_3\text{-}CH(OH)\text{-}COONa \ \text{(acide lactique) chlorite sodique}$$

et

$$5HClO_2 \rightarrow 4ClO_2 \ + \ HCl \ + \ 2H_2O \ \text{dioxyde de chlore}$$

Suivant cette réaction, le dioxyde de chlore est bien libéré au sein de l'appareil.

Or, le dioxyde de chlore a pour particularité d'être un composé bactéricide, virucide et sporicide et d'avoir un pouvoir de fixation très supérieur à celui du chlore natif. Ses propriétés biocides sont moins sensibles au pH que celles du chlore. Ceci se traduit par une conservation de ses propriétés biocides dans une gamme de pH beaucoup plus large que le chlore gazeux.

Pour la réalisation de l'invention, deux produits sont préparés séparément.

Le premier produit, solution base, a une composition centésimale du type qui suit :

| | |
|---|---|
| - chlorite sodique à 30% | 30 à 50% |
| - diéthylène glycol | 1 à 5% |
| - sel trisodique d'acide nitrilotriacétique à 40% | 0,2 à 2% |
| - eau déïonisée | complément à 100% |

La densité du chlorite sodique de 30% à 25°C est de 1,20.

Le second produit, solution activatrice, a la composition centésimale du type suivant :

| | |
|---|---|
| - acide lactique à 80%, en qualité alimentaire | 70 à 90% |
| - eau déïonisée | 30 à 10% |

La densité de l'acide lactique de 80% à 25°C est de 1,18.

Ensuite, l'opérateur effectue le mélange de préférence dans une proportion de la solution activatrice à la solution base de 3:4, c'est-à-dire en additionnant 200 millilitres de la solution base avec 150 millilitres de la solution activatrice.

Le mélange ainsi obtenu est introduit dans l'appareil d'hémodialyse, avec de l'eau courante.

La dilution et la réaction se déroulent pendant le cycle de rinçage de l'appareil d'hémodialyse selon la figure unique.

Or, le cycle de rinçage est précédé par l'évacuation du fluide résiduel du cycle précédent.

Au cours de la phase d'évacuation, l'ouverture simultanée de la valve d'alimentation 1 du dialysat, de la valve de la conduite d'écoulement 2 et des valves 3 solénoïdales à 3 voies, et la mise en marche de la pompe de recirculation 4, permettent l'écoulement du fluide qui reste dans le récipient de préparation 5 et dans l'ensemble des conduites de l'appareil.

Cette phase dure environ 2 minutes.

Puis, la phase de nettoyage et/ou de désinfection proprement dite commence.

Le mélange selon l'invention est préparé comme décrit ci-dessus dans une quantité de 350 millilitres.

4

La dilution de ce mélange dans l'eau courante étant de 1/35, une solution totale de 12,25 litres est introduite en 6 dans l'appareil.

L'introduction de cette solution totale se fait en continu ou en une seule fois.

La valve solénoïdale 7 s'ouvre et lorsque la solution totale formant le produit utilisé comme désinfectant circule dans tout le circuit hydraulique, la phase stricto-sensu de nettoyage-désinfection s'effectue alors, la valve 2 solénoïdale de la conduite d'écoulement se ferme à nouveau, pendant que les autres restent ouvertes au cours du cycle, puis se rouvre à nouveau à intervalles réguliers. Par conséquent, la composition formant le produit désinfectant circule bien dans tout le circuit et assure un nettoyage et une désinfection parfaite du circuit d'écoulement et des valves solénoïdales.

La dilution se fait donc à l'intérieur de la machine et la solution qui en résulte y circule pendant 10 minutes, toujours à la même concentration.

Après 10 minutes, les 12,25 litres du mélange sont passés dans le circuit intérieur de l'appareil car pendant le même temps, un volume équivalent est évacué de l'appareil.

En fait, comme la solution base contient une quantité suffisante de chlorite sodique, 1,8 gramme de chlorite sodique est fourni par minute ainsi que 11,4 grammes d'acide lactique, ce qui synergise la solution.

A la fin de l'opération de nettoyage et/ou de désinfection, au terme de 10 minutes, 18 grammes de chlorite sodique et 114 grammes d'acide lactique ont donc traversé le circuit de l'appareil d'hémodialyse.

Par ailleurs, tout au long de cette phase de nettoyage-désinfection, le pH de la solution est maintenu entre 2,8 et 3,2.

A une telle valeur de pH acide, la dissolution des précipités qui sont restés dans l'appareil après le traitement d'hémodialyse, se réalise facilement. De même, toute trace de vie (micro-organismes, virus, spores) est détruite et l'appareil est alors prêt pour une nouvelle utilisation.

La solution finale selon le procédé décrit ci-dessus, répond aux normes françaises (AFNOR) suivantes :
- antiseptiques et désinfectants utilisés à l'état liquide, miscibles à l'eau et neutralisables, détermination de l'activité bactéricide (méthode par dilution-neutralisation) (T72-150),
- antiseptiques et désinfectants utilisés à l'état liquide, miscibles à l'eau et neutralisables, détermination de l'activité bactéricide en présence de substances interférentes définies (méthode par dilution-neutralisation) (T72-170),
- désinfectants utilisés à l'état liquide miscibles à l'eau, détermination de l'activité antibactérienne pour la décontamination des surfaces (méthode des porte germes) (T72-190),
- antiseptiques et désinfectants utilisés à l'état liquide, miscibles à l'eau et neutralisables, détermination de l'activité fongicide (méthode par dilution-neutralisation) (T72-200).
- antiseptiques et désinfectants utilisés à l'état liquide, miscibles à l'eau et tuant les spores bactériennes, détermination de l'activité sporicide en présence ou non de substances interférentes (méthode par filtration sur membranes) (T72-231),
- procédés de décontamination des surfaces par voie aérienne dans des enceintes closes, détermination du temps d'application (T72-281), et
- antiseptiques et désinfectants utilisés à l'état liquide, miscibles à l'eau, détermination de l'activité virucide vis-à-vis des bactériophages, (T72-181).

De plus, il a été vérifié que la composition telle qu'utilisée dans le procédé selon l'invention, ne provoque aucune corrosion des éléments formant l'appareil d'hémodialyse.

En effet, un essai accéléré a été réalisé, équivalent à 9 000-10 000 heures de fonctionnement, c'est-à-dire 3 ans d'utilisation de l'appareil. On a ainsi déterminé l'attaque superficielle, l'accroissement de masse, les caractéristiques physiques et fonctionnelles de ces matériaux. Or, aucune corrosion importante n'a pu être observée ni sur l'acier inoxydable ni sur le verre, la gomme de silicone, le nylon ou encore le téflon.

Quant aux incrustations tant organiques (protéines de poids moléculairee faible, créatinine, urée, virus) qu'inorganiques (carbonates, urates, phosphates, hydroxydes, ...) elles sont toutes éliminées.

Un appareil d'hémodialyse qui présentait une grande accumulation de dépôts, due à un arrêt prolongé dans le système de désinfection, a été soumis à trois séances de nettoyage, pendant 24 heures suivant le procédé selon l'invention. A chaque cycle de nettoyage, l'efficacité du produit a été vérifiée du fait de la disparition des dépôts visibles sur les surfaces des tubes de cristal de l'appareil.

Ce procédé remplace également avantageusement l'utilisation du formol car il permet de faire disparaître directement tous les dépôts et déchets accumulés par les bactéries, les virus et les champignons.

Ceci est illustré par l'expérience suivante. Deux dispositifs de contrôle de l'hémodialyse qui présentaient une contamination par Pseudomonas Aeruginosa et qui avaient déjà été désinfectés à plusieurs reprises avec des solutions de formol à 1% sans réussir à éliminer cette bactérie, ont été soumis à deux cycles de désinfection de 10 heures chacun selon le procédé selon l'invention, sans interruption.

Après la seconde désinfection, des échantillons ont été prélevés et ont montré l'absence de Pseudomonas Aeruginosa.

Le procédé selon l'invention est aussi très efficace en phase gazeuse.

En résumé, les avantages du procédé selon l'invention découlant des propriétés nettoyantes et/ou désinfectantes de la composition utilisée dans la présente invention, sont exposés dans le tableau ci-dessous en comparaison avec les procédés traditionnellement utilisés.

## – TABLEAU –

| | Elimination des incrustations inorganiques | Elimination des dépôts organiques | Activité contre Pseudomonas Aeruginosa | Activité en phase gazeuse |
|---|---|---|---|---|
| Procédé selon l'invention | + + + | + + + | + + + | + + + |
| Procédé utilisant 50g de chlore natif par litre | - - - | + + + | + - - | + - - |
| Procédé utilisant le formol | - - - | - - - | + - - | + + + |
| Procédé utilisant l'acide acétique | + + + | + - - | + - - | - - - |

Dans ces circonstances, aussi bien en phase liquide que gazeuse, la composition utilisée dans le procédé a de bonnes propriétés bactéricides qui, associées aux propriétés nettoyantes, lui apportent des avantages et une efficacité bien supérieurs aux autres produits utilisés dans les procédés connus de la technique.

De ce fait, la désincrustation périodique (hebdomadaire) par des solutions acides et la stérilisation au moyen du formol (une fois par mois) ne sont plus nécessaires.

Donc, en une seule opération, le procédé selon l'invention peut remplacer toutes les différentes phases de nettoyage et/ou de désinfection connues dans l'art, pour les appareils d'hémodialyse.

Ce procédé très efficace est également simple et pratique d'emploi et permet un gain de temps considérable dans les services de néphrologie. Enfin, tout danger encouru par la stérilisation au formol pour les patients et le personnel des hôpitaux, est écarté.

**Revendications**

1. Procédé pour nettoyer, désinfecter et stériliser des appareils d'hémodialyse ayant un circuit d'hémodialyse, le nettoyage précité comprenant la dispersion des dépôts organiques et la dissolution des précipités inorganiques sur les surfaces internes du circuit d'hémodialyse, caractérisé en ce qu'il consiste à :
   - mélanger une composition basique comprenant l'ion chlorite avec une composition acide comprenant l'acide lactique, dans un rapport de la composition acide à la composition basique allant de 1:2 à 3:4,
   - introduire le mélange ainsi obtenu additionné d'eau dans l'appareil d'hémodialyse, le rapport du mélange à l'eau étant d'environ 1:34, pour former une solution finale acide, et
   - faire circuler la solution finale dans l'appareil, où le mélange est dilué dans l'eau et où la composition basique réagit avec la composition acide selon les formules suivantes :

   $$CH_3\text{-}CH(OH)\text{-}COOH + ClO_2{}^- \rightarrow HClO_2 + CH_3\text{-}CH(OH)\text{-}COO^-$$

   et

   $$5HClO_2 \rightarrow 4ClO_2 + HCl + 2H_2O$$

2. Procédé selon la revendication 1, caractérisé en ce que la composition basique contient 30 à 50% en masse de chlorite de sodium et 70 à 50% en masse d'eau déïonisée.

3. Procédé selon la revendication 1 ou 2, caractérisé en ce que la composition acide contient 70 à 90% en masse d'acide lactique et 30 à 10% en masse d'eau déïonisée.

4. Procédé selon l'une des revendications précédentes, caractérisé en ce que le mélange est introduit en continu ou en une seule fois dans l'appareil d'hémodialyse.

5. Procédé selon l'une des revendications précédentes, caractérisé en ce que la solution finale circule pendant 10 minutes dans l'appareil.

**Claims**

1. Method of cleaning, disinfecting and sterilizing haemodialysis apparatus having a haemodialysis circuit, the aforesaid cleaning comprising the dispersion of the organic deposits and the dissolving of the inorganic precipitates upon the internal surfaces of the haemodialysis circuit, characterized in that it consists in :
   - mixing a basic composition comprising the chlorite ion with an acid composition comprising the lactic acid in a ratio of the acid composition to the basic composition ranging from 1:2 to 3:4,
   - introducing the mixture thus obtained and admixed with water into the haemodialysis apparatus, the mixing ratio to water being about 1:34 to form a final acid solution and
   - circulating the final solution in the apparatus wherein the mixture is diluted in the water and wherein the basic composition reacts with the acid composition according to the following formulae :

$$CH_3\text{-}CH(OH)\text{-}COOH + ClO_2{}^-\rightarrow HClO_2 + CH_3\text{-}CH(OH)\text{-}COO\text{-}$$

and

$$5HClO_2\rightarrow 4ClO_2 + HCl + 2H_2O$$

2. Method according to claim 1, characterized in that the basic composition contains 30 to 50 % by mass of sodium chlorite and 70 to 50 % by mass of deionized water.

3. Method according to claim 1 or 2, characterized in that the acid composition contains 70 to 90 % by mass of lactic acid and 30 to 10 % by mass of deionized water.

4. Method according to one of the foregoing claims, characterized in that the mixture is inserted continuously or in one single time into the haemodialysis apparatus.

5. Method according to one of the foregoing claims, characterized in that the final solution circulates for 10 minutes in the apparatus.

**Patentansprüche**

1. Verfahren zum Reinigen, Desinfizieren und Entkeimen von einen Hämodialysekreislauf aufweisenden Hämodialysegeräten, wobei die vorgenannte Reinigung die Dispersion der organischen Ablagerungen und die Auflösung der anorganischen Niederschlägen auf den Innenflächen des Hämodialysekreislaufes umfasst, dadurch gekennzeichnet, dass es darin besteht:
   - eine das Chlorit-Ion umfassende basische Verbindung mit einer die Milchsäure umfassenden sauren Verbindung in einem Verhältnis der sauren Verbindung zur basischen Verbindung von 1:2 bis 3:4 zu vermischen,
   - das somit erhaltene mit Wasser versetzte Gemisch in das Hämodialysegerät einzuführen, wobei das Verhältnis des Gemisches zum Wasser ungefähr 1:34 beträgt, um eine saure Endlösung zu bilden, und
   - die Endlösung im Gerät umlaufen zu lassen, wo das Gemisch in dem Wasser verdünnt wird und wo die basische Verbindung mit der sauren Verbindung gemäss den folgenden Formeln reagirt:

$$CH_3\text{-}CH(OH)\text{-}COOH + ClO_2\text{-}\rightarrow HClO_2 + CH_3\text{-}CH(OH)\text{-}COO\text{-}$$

und

$$5HClO_2\rightarrow 4ClO_2 + HCl + 2H_2O$$

2. Verfahren nach Anspruch 1, dadurch gekennzeichnet, dass die basische Verbindung 30 bis 50 Massenprozent an Natriumchlorit und 70 bis 50 Massenprozent an entionisiertem Wasser enthält.

3. Verfahren nach Anspruch 1 oder 2, dadurch gekennzeichnet dass die saure Verbindung 70 bis 90 Massenprozent an Milchsäure und 30 bis 10 Massenprozent an entionisiertem Wasser enthält.

4. Verfahren nach einem der vorangehenden Ansprüche, dadurch gekennzeichnet, dass das Gemisch kontinuirlich oder in einem Mal in das Hämodialysegerät eingeführt wird.

5. Verfahren nach einem der vorangehenden Ansprüche, , dadurch gekennzeichnet, dass die Endlösung während 10 Minuten in dem Gerät umläuft.